# EUROPEAN PATENT SPECIFICATION

(11) **EP 1 094 316 B1**
(45) Date of publication and mention of the grant of the patent: **28.12.2005**
(21) Application number: 00123005.1
(22) Date of filing: 23.10.2000
(51) Int. Cl.: G01N 33/50, G01N 33/53, G01N 33/574

(54) **Method for the detection of DNA replicating cells**
Verfahren zum Nachweis DNS-replizierender Zellen
Méthode pour la détection des cellules avec l'ADN réplicant

(30) Priority: 22.10.1999 EP 99120820
(43) Date of publication of application: 25.04.2001
(73) Proprietor: Hammers, Hans-Jörg, MC Lean,VA 22102 (US); Schlenke, Peter, 23627 Gross Grönau (DE)
(72) Inventor: Hammers, Hans-Jörg, MC Lean,VA 22102 (US); Schlenke, Peter, 23627 Gross Grönau (DE)
(74) Representative: Vossius & Partner

(56) References cited:
- US-A- 5 747 258
- DATABASE BIOSIS [Online] BIOSCIENCES INFORMATION SERVICE, PHILADELPHIA, PA, US; 1994 LI XUN ET AL: "Detection of 5-bromo-2-deoxyuridine incorporated into DNA by labeling strand breaks induced by photolysis (SBIP)." Database accession no. PREV199598125105 XP002183106 & INTERNATIONAL JOURNAL OF ONCOLOGY, vol. 4, no. 6, 1994, pages 1157-1161, ISSN: 1019-6439
- DATABASE BIOSIS [Online] BIOSCIENCES INFORMATION SERVICE, PHILADELPHIA, PA, US; 1992 TOBA K ET AL: "IMPROVED STAINING METHOD FOR THE SIMULTANEOUS FLOW CYTOFLUOROMETRIC ANALYSIS OF DNA CONTENT S-PHASE FRACTION AND SURFACE PHENOTYPE USING SINGLE LASER INSTRUMENTATION" Database accession no. PREV199293052814 XP002183107 & CYTOMETRY, vol. 13, no. 1, 1992, pages 60-67, ISSN: 0196-4763
- DATABASE BIOSIS [Online] BIOSCIENCES INFORMATION SERVICE, PHILADELPHIA, PA, US; 1 August 2000 (2000-08-01) HAMMERS H J ET AL: "Ultraviolet-induced detection of halogenated pyrimidines: Simultaneous analysis of DNA replication and cellular markers." Database accession no. PREV200000478998 XP002183108 & CYTOMETRY, vol. 40, no. 4, 1 August 2000 (2000-08-01), pages 327-335, ISSN: 0196-4763

## Description

The present invention relates to a method for identifying DNA replicating cells, which method comprises the steps of adding during the cultivation of cells a halogenated pyrimidine to the medium, preferably BrdUrd, treating the harvested cells by UV irradiation, fixing the cells prior to or after UV treatment, resuspending the cells in a solution with reduced ionic strength and identifying cells with the incorporated halogenated pyrimidine, e.g. by using labelled antibodies. The method of the present invention can be combined with the simultaneous detection of cellular markers, e.g. surface antigens. Thus, the method of the present invention is useful for the analysis of apoptosis, proliferation and cell-cycle kinetics as well as the diagnosis and prognosis of malignancies.

The current concept of cancerogenesis is based on the appearance of multiple genetic accidents in somatic cells resulting in autonomous cell proliferation and metastasis. Chromosomal aberrations frequently occur in cancer cells which lead to DNA content changes and uncontrolled tumor cell growth in solid tumors and hematological malignancies. Cell cycle analysis are well established for characterizing the proliferative potential of tumor cells. For this end, the thymidine analogue 5-bromo-2-deoxyuridine (BrdUrd) and ³H-thymidine are the golden standards in identifying DNA synthesizing (replicating) cells. Today, BrdUrd has widely replaced the radioactive and time-consuming ³H-thymidine, and its widespread application in cell-cycle analysis of malignant tumors (1, 2), proliferating tissues, immunological responses (3) and pharmacological studies has been reviewed extensively (4, 5, 6). Besides determination of the S-phase fraction (SPF), other aspects of proliferation such as labeling index (LI), duration of S-phase (Ts) and potential doubling time (Tpot) can be derived (7, 8). This feature makes BrdUrd a versatile tool which provides not only static, but also kinetic information about cycling cells. In clinical studies SPF, LI and Tpot have been found to be important prognostic factors in various human malignancies such as lung cancer (9), Non-Hodgkin lymphoma (10), glioblastoma multiform (11) and multiple myeloma (12, 13, 14).

After BrdUrd incorporation into DNA monoclonal antibodies (mAbs) allow for sensitive identification and enumeration of DNA synthesizing cells, i.e. S-phase cells (15). Counterstained with stoichiometric binding DNA dyes such as propidium iodide (PI) or 7-aminoactinomycin D (7-AAD), S-phase cells and cells in G_{0/1} and G₂/M-phase can be discriminated (16). A simultaneous flow cytometric analysis of cell surface antigens, BrdUrd incorporation into DNA, and DNA quantification with 7-AAD, using an hypotonic buffer after cell fixation is described in Toba et al., Cytometry (1992)13:60-67. However, the conventional immunological detection of BrdUrd with mAbs requires partially denaturated, single stranded DNA to expose the epitope. Usually, harsh physical conditions such as heat (>90°C) or acid (2-4N) treatment are required making it difficult to combine BrdUrd detection with the analysis of other cellular markers such as immunophenotyping or apoptosis. Since the introduction of the BrdUrd technique in 1982 by Gratzner et al. (15) several alternative, milder denaturation or detection procedures have been developed. These techniques employ enzymes to partially digest DNA (Exonuclease III, DNAse I) (17- 20) or label strand breaks induced by ultraviolet (UV) light photolysis with a subsequent terminal-deoxynucleotidyl-transferase (TdT) reaction (SBIP) (21). However, these methods exhibit a number of disadvantages. The Exonuclease III approach leads to cell loss and clumping (22) and the DNAse I can lack sensitivity (23), cause overdigestion, artifacts or a high coefficient of variation (CV) in the DNA histogram (22). SBIP requires the exclusion or discrimination of apoptotic events as it labels SSB induced by photolysis and apoptotic DNA at the same time. (21, 24). Thus, these methods are rather complex and time consuming. Moreover, the reliable and quantitative detection of DNA synthesing cells, i.e. S-phase cells, by the use of the methods of the prior art is difficult.

Thus, the technical problem underlying the present invention is to provide a method for the detection of DNA replicating cells which overcome the disadvantages of the methods of the prior art, e.g. (i) allows the simultaneous detection of DNA replication and other cellular markers of interest (without enzymatic treatment), (ii) avoids artifacts and (iii) reduces costs and preparation time.

The solution to the above technical problem is achieved by providing the embodiments characterized in the claims. A novel methodological principle using ultraviolet induced detection of halogenated pyrimidines in solution with reduced ionic strengths had been developed which allows for simultaneous analysis of cell proliferation, immunophenotyping and other cellular markers such as apoptosis. The method of the present invention identifies DNA replicating cells by their incorporation of halogenated pyrimidines, e.g. 5-bromo-deoxyuridine or 5-iodo-deoxyuridine. Irradiation with UV light, e.g. UV-C, UV-B or UV-A plus, e.g., Hoechst 33258, and subsequent treatment with a hypotonic buffer makes the pyrimidine accessible to specific ligands, e.g. monoclonal antibodies allowing its sensitive detection. Inspite of previous approaches the method of the present invention lacks the need for any enzymatic treatment such as DNA digestion or strand-break labeling after UV irradiation making the method fast, inexpensive and convenient to use. Mild fixation procedures with a wide variety of fixatives, e.g. crosslinking aldehydes and coagulative fixatives such as alcohols and metal salts, can be applied giving the opportunity for simultaneous detection of a wide range of cellular markers e.g. surface molecules, cytosceletal proteins or apoptosis. Fixation before or after UV-irradiation is possible and allows adjustments in individual laboratories. Extensive crosslinking with aldehydes, i.e. 30 min of 1% PFA at RT, however, stabilizes the chromatin and prevents subsequent BrdUrd detection, stressing the importance of structural changes in solutions with reduced ionic strength. The successful identification of CD34+, CD138+ or CD19+ cells out of heterogeneous cell suspensions and their cell-cycle analysis is described in the following Examples. Results correlated very well with acid denaturation (r = 0.938). The average CV of G1 in the DNA histogram was smaller than 5% resulting in good preservation of DNA distribution. The signal-to-noise ratio was almost twice as high for as for acid denaturation allowing convenient discrimination of BrdUrd positive cells.

In conclusion, a new technology for S-phase specific cell cycle analysis in heterogenous cell suspensions on the basis of ultraviolet induced BrdU detection is provided. One of the most appealing aspects of the method of the invention is its simple, inexpensive and rapid procedure. Unlike the enzymatic approaches, which need multiple steps of fixation, washing, digestion or labeling, the method of the invention requires only a short period of UV irradiation (e.g. 5 min), a short fixation period and resuspension in a solution with reduced ionic strength containing, e.g. an anti-BrdUrd-mAb for detection of the S-phase fraction and if desired, e.g., 7-AAD as a counterstaining dye for DNA content analysis. The mild treatment and infrequent washing steps allows for simultaneous and sensitive detection of BrdUrd in combination with preserved staining of surface molecules, apoptosis and other cellular markers, while avoiding excessive loss of cells or selective enrichment of subpopulations. The advantages of this simplified approach might contribute to encourage the broader use of halogenated pyrimidines and their unique features regarding cell-cycle analysis and cell kinetics in research and clinical studies to further the understanding of proliferation, apotosis and differentiation in human malignancies and other application fields such as basic immunology and drug cytotoxicity testing

Thus, in a first embodiment the present invention relates to a method for identifying DNA replicating cells, which method comprises the following steps: (a) adding during the cultivation of cells a halogenated pyrimidine, (b) treating the harvested cells by UV irradiation, (c) fixing the cells prior to or after UV-irradiation (d) resuspending the cells in a solution with reduced ionic strength and (e) identifying cells with incorporated halogenated pyrimidine.

### LEGENDS TO THE FIGURES:

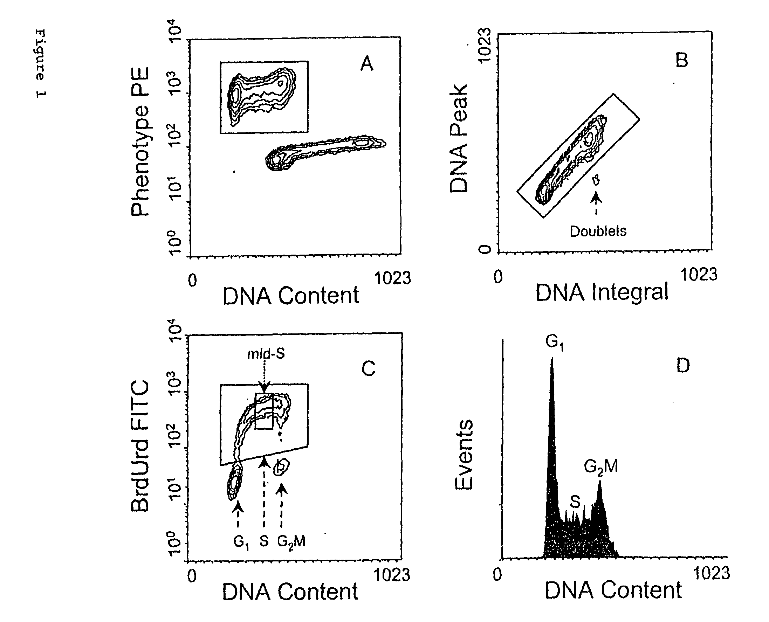

### Fig. 1: Representative cell cycle analysis of a heterogeneous cell suspension.

Raji and Molt-4 cells were mixed and incubated for 60 min with 30 µM BrdUrd at 37°C and subjected to 5 min UV-B irradiation. Before fixation Raji cells were stained with a PE-conjugated anti-CD19 mAB to allow separate analysis of the cell lines (A). After 0.25% PFA fixation for 15min at RT cells were resuspended in a hypotonic sodium borate buffer, anti-BrdUrd FITC mAB was added and DNA counterstained with 7-AAD. (B) Doublets were excluded by there characteristic properties in a DNA Peak versus Integral histogram. (C) All cells with intermediate DNA content (between 2C, i.e. G₁, and 4C, i.e. G₂/M) are stained for BrdUrd positive and can be identified easily. To assess sensitivity a signal-to-noise ratio (S/N-ratio) given by the ratio of mean fluorescent intensities of mid-S phase cells to cells in G1 G_{0/1} was determined. (D) Corresponding DNA-content histogram with good preserved distribution properties. (A) Note that Raji and Molt-4 not only differ by CD19 expression, but also by DNA content confirming the complete discrimination of both cell lines by surface markers without any hints of redistribution phenomena.

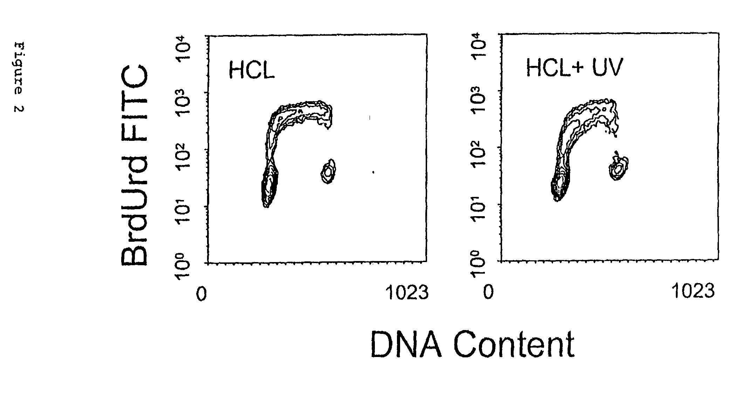

### Fig. 2: Residual BrdUrd after UV irradiation.

HS-Sultan cells were exposed to 30 µM BrdUrd for 1 hour and divided. Half of the cells underwent classical 2N HCl denaturation for 20 min at RT. The other half was irradiated with UV-B light for 5 min and then subjected to HCl denaturation as well. BrdUrd was only partially photolysed as there was a slight decrease in BrdUrd signal intensity in comparison to non-irradiated cells.

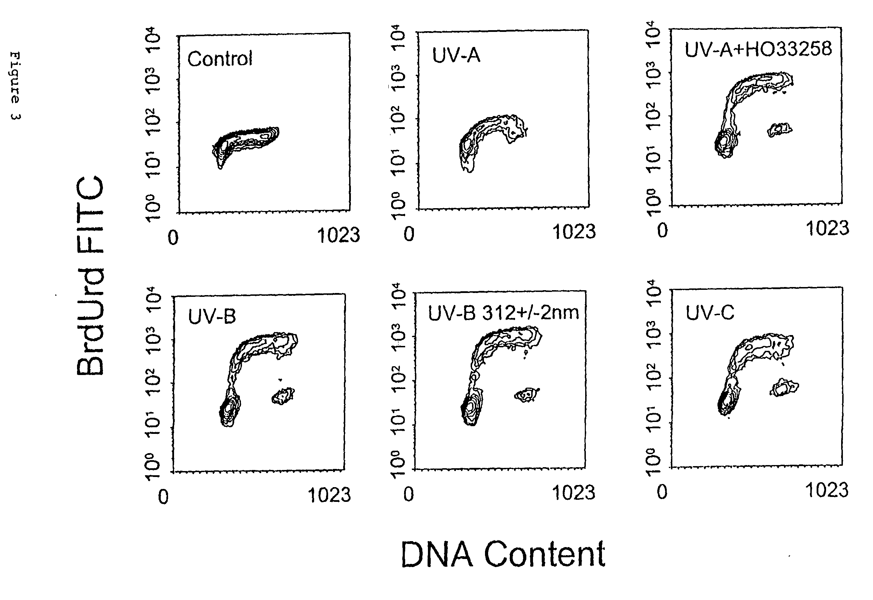

### Fig. 3: BrdUrd incubated HL 60 cells were irradiated with different spectra of ultraviolet light and subjected to the method of the invention.

HL60 cells have been incubated with 30 µM BrdUrd for 1 hour. Cells in (A) did not undergo UV irradiation. (B) Cells were irradiated with UV-A (340-380 nm), (C) cells were irradiated with UV-A in the presence of 10 µg/ml Hoechst 33258. (D) Cells were exposed to a UV Handlamp emitting predominantly UV-B (280-320 nm), (E) irradiated with ultraselective UV-B (311±2 nm), (F) conventional Hood-lamp emitting mainly UV-C (254 nm). UV-B and with instances UV-C are able to induce BrdUrd detection directly, whereas UV-A is only efficient in the presence of bisbenzimides such as Hoechst dye 33258. Cells were irradiated 5 min regardless of its source.

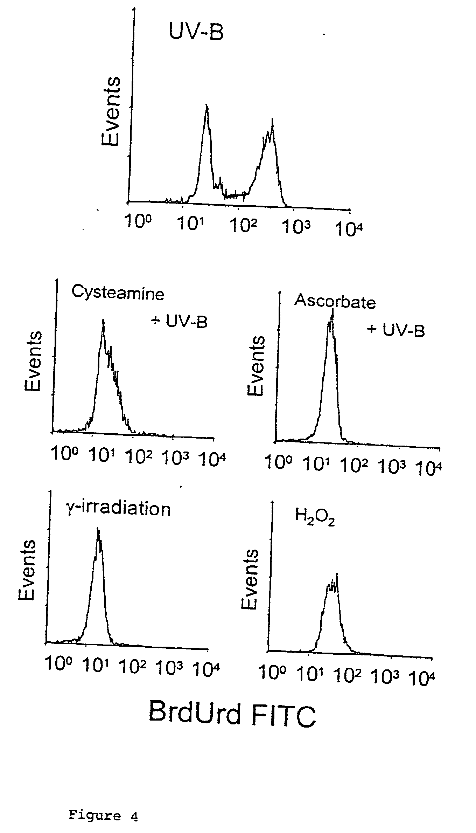

### Fig. 4: Influence of antioxidants and single-strand break (SSB) / double strand break (DSB) inducers.

HL-60 cells were incubated with 30 µM BrdUrd for 60 min and split for comparison. Cells were irradiated with UV-B, UV-B in the presence of 20 mM cysteamine or 20 mM sodium ascorbate. In HL-60 cells the addition of antioxidants and radical scavenger cysteamine and sodium ascorbate lead to almost complete abrogation of BrdUrd detection. To elucidate the efficacy of other strand-break inducers, cells were irradiated with 30 Gy Cs¹³⁷ γ-radiation or treated with 10mM H₂O₂ for 20 min at RT. Interestingly, SSB / DSB inducing γ-irradiation and SSB inducing H₂O₂ treatment did not allow BrdUrd detection suggesting a different quality and/or amount of DNA damage in comparison to UV irradiation.

### Fig. 5: Requirement of hypotonicity for BrdUrd detection.

Cells were exposed to UV-B, fixed and then subjected to either isotonic phosphate-buffered saline (PBS) (300 mosm) or a hypotonic PBS/sodium borate buffer (68 mosm). Only cells resuspended in a buffer with reduced ionic strength were amenable to BrdUrd detection.

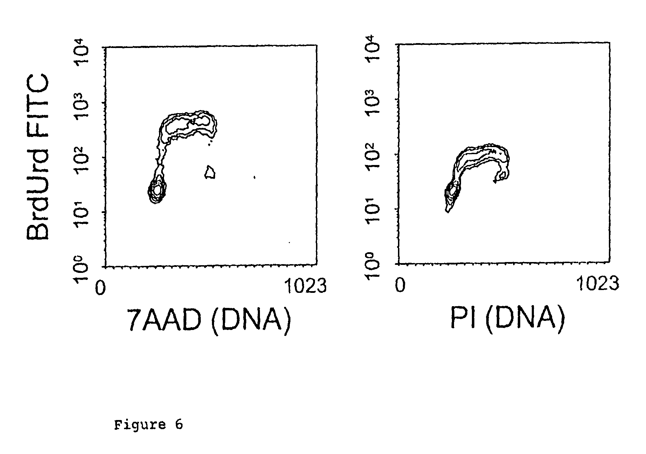

### Fig. 6: Quenching effect of Propidiumiodide (PI).

Cells were incubated with BrdUrd and treated according to the method of the invention. Cells were split and stained with PI or 7-Aminoactinomycin D (7-AAD). The unspecific intercalating DNA dye propidium iodide (PI) quenches the BrdUrd detection in S-phase cells to a larger degree, whereas the GC-specific intercalator 7-aminoactinomycin D (7-AAD) leaves the FITC fluorescence untouched suggesting a new binding mode of the anti-BrdUrd-mAB.

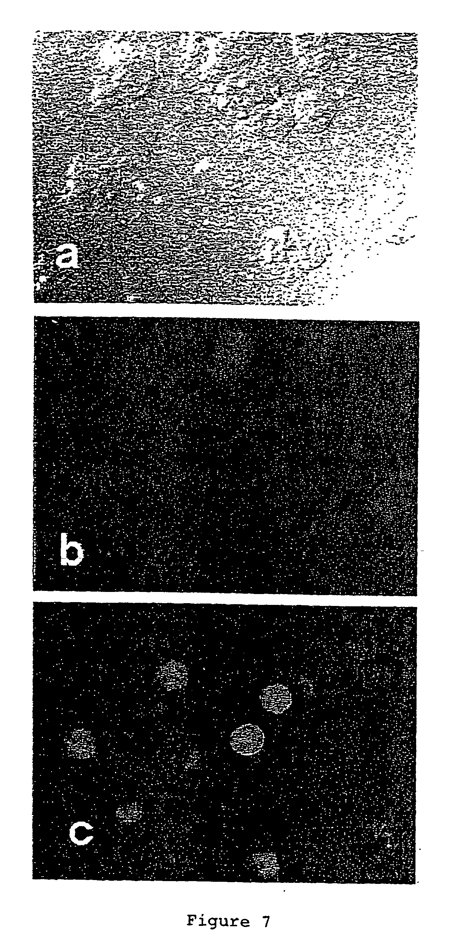

### Fig. 7: Immunofluorescence microscopy.

Raji cells were incubated with 30 µM BrdUrd for 1 hr and prepared according to the method of the present invention. Cells were carefully spun down and analyzed under an immunofluorescence microscope. Cells were identified with transmitted light interference first (a) and then illuminated with blue light to analyze for BrdUrd staining (b). Note positive and negative nuclei by comparing (a) with (b). (c) Cells were counterstained with 7-AAD, a dark red DNA dye.

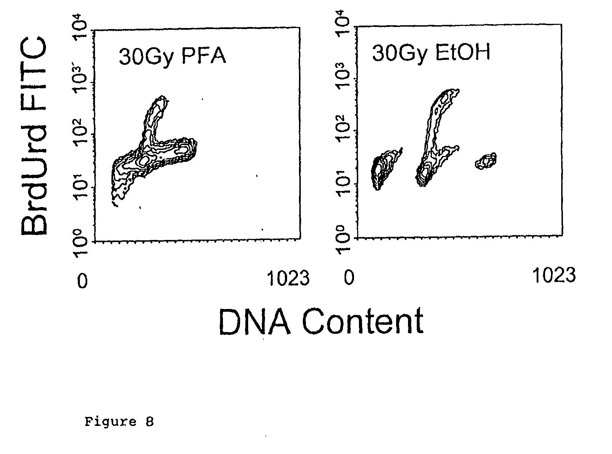

### Fig. 8: Simultaneous cell cycle analysis and detection of apoptosis.

Exponentially growing Raji cells were irradiated with 30 Gy Cs¹³⁷ γ-irradiation and incubated for additional 4 hrs at 37°C. For the last 60 min BrdUrd in a final concentration of 30 µM was added. Cells were then subjected to the method of the invention either with PFA as a cross-linking or ethanol as a coagulative fixative. Note that depending on the fixation procedure apoptotic low molecular weight DNA can be extracted identifying apoptotic cells next to DNA replication.

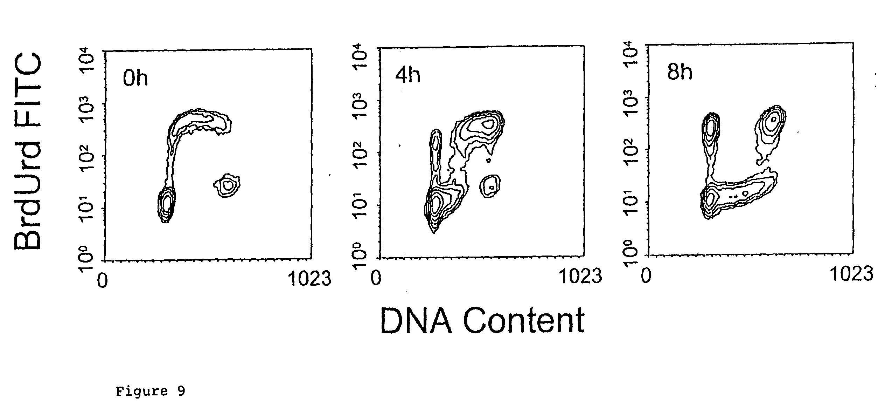

### Fig. 9: Pulse-Chase labeling experiment.

HS-Sultan cells were pulse-labelled for 40 min with 30 µM BrdUrd, washed with warm medium and allowed to continue growing at 37°C. Cells were removed from cell culture at 0, 4 and 8hrs and subjected to the method of the invention. Labelled divided and undivided cells can be identified easily and kinetic data can be obtained.

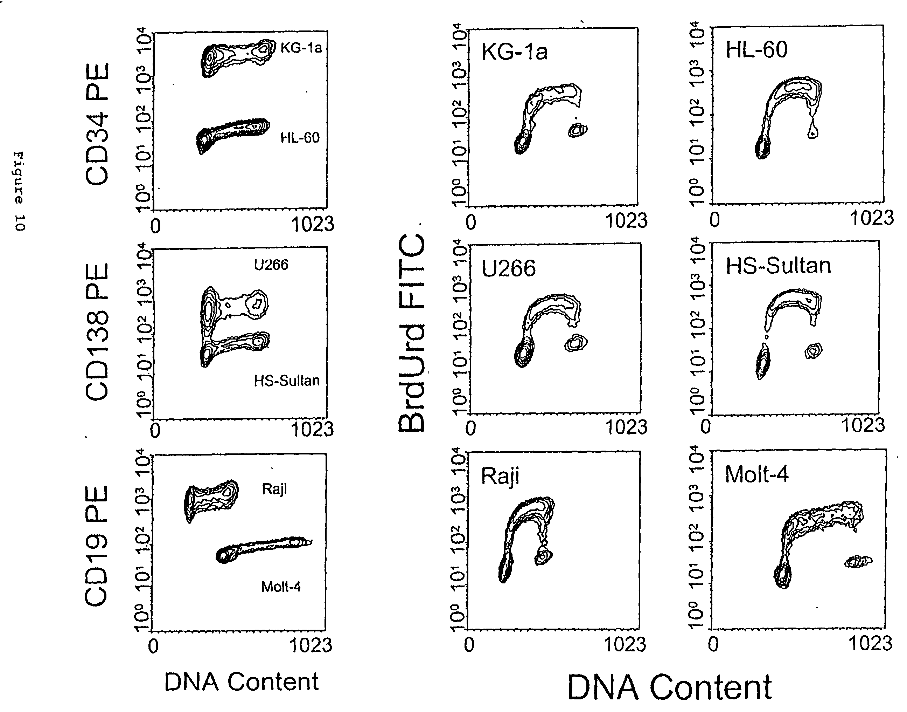

### Fig. 10: Cell-cycle analysis and simultaneous phenotyping.

Three pairs of two cell lines with distinctive surface markers were formed and mixed: HL-60 with KG-1a bearing CD34, HS-Sultan with U266 expressing CD138 and Molt-4 with Raji, a CD19 positive cell line. Cells were exposed to 30 µM BrdUrd for 1 hr and irradiated with UV-B for 5 min. Surface staining was carried out before fixation with 0.25% PFA for 15 min at RT. Cells were subjected to a hypotonic PBS/sodium tetraborate buffer and anti-BrdUrd FITC and 7-AAD were added. Cells were allowed to incubate at least one hour at RT before measurement. In all suspensions both cell lines could be discriminated by their expression of surface markers and by appropriate gating the cell-cycle of each cell line could be analyzed. The obtained data were correlated with results from acid denaturation.

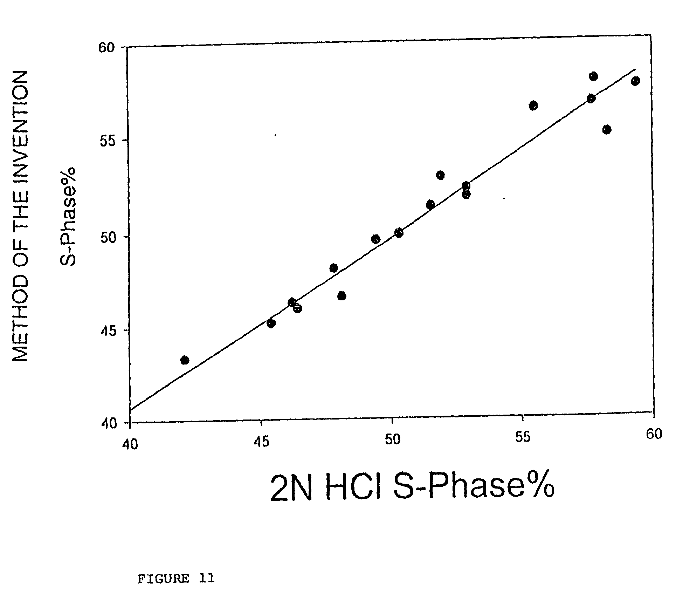

### Fig. 11: Correlation of S-Phase fraction determined by the method of the invention and acid denaturation, respectively.

Six different cell lines KG1a, HL60, U266, HS-Sultan, Raji and Molt-4 were incubated for 1hr with 30 µM BrdUrd and subjected parallel to the method of the invention and acid denaturation with 2 N HCl, respectively. These experiments were repeated three times for each cell line. S-phase fractions correlated well with a coefficient of r = 0.938.

The method of the invention can be applied for any cells, preferably animal cells and most preferably mammalian cells, e.g. human cells. The cells are cultivated according to standard procedures and using standard media well known to the person skilled in the art, e.g. according to the method describe in the above Example 1. Furthermore, the method of the invention might be also applicable on unfixed or coagulative fixed tissue sections after in vivo or in vitro BrdUrd incorporation.

In order to be able to analyze the cell cycle, e.g. for the detection of S-phase cells the halogenated pyrimidine should be added to the culture, preferably at a cell density of 1 x 10⁴ - 2 x 10⁶ cells/ml. The concentration of the halogenated pyrimidine should be in the range of 1µM to 100µM., preferred are final concentrations of the halogenated pyrimidine in the range of 10 to 30 µM. After addition of the pyrimidine the cells are further cultivated for a sufficient period of time in order to allow the incorporation of the pyrimidine into the genome of the cells, e.g. for 10 to 120 min depending on the cell system, preferably about 60 min.

Halogenated pyrimidines useful in the method of the invention are well known to the person skilled in the art and can be obtained from commercial sources. The halogenated pyrimidine useful in the method of the present invention is a halogenated thymidine analogue. Preferred are the following halogenated thymidine analogues: 5-bromo-2-deoxyuridine (BrdUrd), 5-iodo-2-deoxyuridine (IdUrd), 5-fluoro-2-deoxyuridine (FdUrd) or 5-chloro-2-deoxyuridine (CldUrd). The most preferred thymidine analogue is 5-bromo-2-deoxyuridine (BrdUrd). These analogues can comprise additional chemical modifications which do not adversely affect the properties of the analogue with respect to the method of the invention, e.g. its capability of being incorporated into DNA, being prone to photolysis, being detectable, e.g. by specific antibodies etc.

After cultivation the cells are irradiated with UV light, preferably after harvesting the cells by centrifugation and resuspending in a suitable medium, e.g. phosphate-buffered saline or isotonic sodium chloride. The irradiation is carried out with ultraviolet light, namely UV-A, UV-B or UV-C light, preferably with UV-A or UV-B. Suitable periods of irradiation can be easily determined by the person skilled in the art and are, for example, in the range of 1 to 30 min. e.g. for the Herolab UV-B source 8W, 1cm distance between UV-light and cell suspension, 3 to 10 min irradiation. More preferred is a irradiation with UV-B light with a wavelength in the range between 280 to 320 nm. Most preferred is a wavelength in the range between 309 and 313 nm. Preferably, when using UV-A light in the method of the invention the irradiation is combined with the use of A-T specific DNA dyes, preferably bisbenzimides, e.g. Hoechst dye 33258 in order to achieve the best results. Preferably the irradiation is carried out in the presence of 1 to 50 µg/ml Hoechst dye 33258. If additional DNA content analysis is desired, counterstaining with stochiometrically binding DNA dyes, such as 7-amino-actinomycin (7-AAD) is the preferred for multi-color flow cytometry on single argon laser platforms due to its high stoke shift and small spectral overlap with PE conjugates (50, 51).

Before resuspending the cells in a solution with reduced ionic strength fixation of the cells is carried. This fixation can be carried out prior to or after UV irradiation. Suitable fixation methods and fixatives are well known to the person skilled in the art and additionally described in the following examples. Mild fixation procedures with a wide variety of fixatives, e.g. crosslinking fixatives, such as aldehydes, imidates or dimidates, carbodiimides, diisocyanates, diimido esters, diethylpyrocarbonate, maleimides or bis maleimides, bezoquinone and coagulative fixatives such as alcohols and metal salts, are preferred allowing the simultaneous detection of a wide range of cellular markers e.g. surface molecules, cytoskeletal proteins or apoptosis.

After irradiation with UV light the cells are resuspended in a solution with reduced ionic strength. The term "solution with reduced ionic strength" as used herein denotes a solution with a ionical osmolality less than 300mosm (e.g., 150meq Na+) and must be in a range allowing the exposition of the pyrimidine incorporated into the DNA to the directly or indirectly labelled ligand, e.g. a specific antibody. Preferably the osmolarity of the solution with reduced ionic strength is less than 200 mosm. More preferred are solutions with an osmolarity in the range between 50 and 100 mosm, e.g. about 68 mosm.

DNA synthesizing (replicating) cells, i.e. S-phase cells, are identified in step (e) of the method of the invention by the determination of the BrdUrd incorporated into the genome of the cells, which is accessible to specific ligands due to the previous treatment with a solution with reduced ionic strength. The ligands are directly or indirectly labelled. Suitable specific ligands include monoclonal or polyclonal antibodies, preferably the ligand is a monoclonal antibody, e.g. an anti-BrdUrd-antibody, which is fluorochrome-conjugated, preferably with fluoresceinisothiocyanate (FITC). Such antibodies can be obtained by routine procedures well known to the person skilled in the art by using the halogenated pyrimidine as an immunogene. The term "antibody" as used herein also comprises a fragment of such an antibody, e.g., a f(ab) or f(ab)₂ fragment, which also specifically recognizes the halogenated pyrimidine, e.g. BrdUrd. Preferably, the monoclonal antibody useful in the method of the invention is a FITC-conjugated antibody.

In a particular preferred embodiment of the method of the invention the cells with the halogenated pyrimidine incorporated into the genome are identified by flow cytometry, immunofluorescence microscopy, confocal microscopy and laser-scan microscopy after binding of the specific ligand to the base analogue, e.g. as described in Example 1.

One of the most important characteristics of the method of the present invention is that surface markers of the cells are not destroyed by the UV-irradiation, thus, e.g. different cell types in heterogeneous cell suspensions can be simultaneously analyzed, for example as described in Examples 1, 7 and 9. Thus, a further preferred embodiment of the method of the present invention comprises the simultaneous detection of at least one cellular marker such as surface molecules, cytoskeletal proteins, nuclear antigens or apoptosis markers, preferably by immunophenotyping according to well known methods, e.g. the methods described in the following Examples. In general, for the simultaneous analysis of cellular antigens the cells are fixed before or after the UV treatment by standard reactions using mild fixatives like crosslinking aldehydes or coagulative alcohols, e.g. paraformaldehyde or ethanol, e.g. 70% Ethanol.

Finally, the method of the present invention is useful for analyzing apoptosis or proliferation (e.g. according to the protocol of Example 7) or for studying cell-cycle kinetics, e.g. by performing pulse-chase experiments as shown in Example 8. The combined analysis of the state of a cell as regards DNA replication, apoptosis and/or cell-cycle kinetics in myeloma patients provides valuable prognostic information, e.g. in multiple myeloma patients a labeling index > 2% is correlated with a poor prognosis. Thus, the method of the present invention is also useful for diagnosing or prognosing a malignancy, benign disease, immunological disorder or infectious disease.

The following Examples illustrate the invention.

### Example 1: General procedures

### (A) CELL LINES, CELL CULTURE AND IMMUNOPHENOTYPING

For the experiments six different human cell lines were used: KG-1a, an acute myeologenous CD34+ leukemia, HL-60, an acute promyelocytic leukemia, U-266, a CD138+ (B-B4) multiple myeloma, HS-Sultan, a Burkitt's lymphoma derived cell line, Raji, a CD19+ B-lymphoblastic EBV+ cell line and MOLT-4, a tetraploid T-lymphoblastic leukemia (ATCC). HL-60, HS-Sultan and Raji cells were cultured in RPMI 1640 medium (Seromed, Biochrom KG, Berlin, Germany) supplemented with 10% fetal calf serum (FCS), 2% L-glutamine, 1% streptomycin/penicillin and KG-1a, U-266 and MOLT-4 cells in IMDM (Bio Whittaker, Verviers, Belgium) containing 20% FCS, 2% L-glutamine and 1% streptomycin/penicillin. Cells were grown to a maximum density of 5 x 10⁵ cells/ml. BrdUrd (Sigma, Deisenhofen, Germany) was added to a final concentration of 30 µM and cell lines were incubated for 60 min in a 37°C incubator with 5% CO₂.

For the correlation experiments three pairs of two cell lines were formed: KG-1a cells were mixed with HL-60 cells, U-266 cells with HS-Sultan cells and Raji cells with Molt-4 cells, respectively. To allow separation by immunophenotype in each pair the surface of one cell line was labelled with a PE-conjugated monoclonal antibody: KG-1a cells with anti-CD34 (clone HPCA-2, Becton-Dickinson, Heidelberg, Germany), U-266 cells with anti-CD138 (clone B-B4, DPC Biermann GmbH, Bad Nauheim, Germany) and Raji cells with anti-CD19 (clone B43, Pharmingen, Hamburg, Germany). Concentrations were used as recommended by the manufacturers' guidelines and cells were incubated at room temperature (RT) in the dark for 20 min. These experiments were carried out three times to generate data for the correlation and comparison with those obtained by acid denaturation.

For the induction of apoptosis HL-60 cells were irradiated with 30 Gy Cs¹³⁷ γ-irradiation. Cells were allowed to continue growing for 3 h at 37°C. BrdUrd was then added and the cells were incubated for another 60 min until harvested.

### (B) HCl denaturation

For validation of the method of the invention and comparison of the results HCl denaturation was chosen as the reference method. Briefly, each cell line was fixed separately in -20°C cold 70% ethanol for about 1h. DNA was partially denaturated in 2N HCl for 20min at room temperature (RT). The acid was carefully neutralized by incubating cells with 0.1M sodium tetraborate (pH 9) for 5 min. Cells were washed once with phosphate-buffered-saline (PBS). Following the manufacturers' recommendations, 20 µl of FITC conjugated anti-BrdUrd (clone 3D4, Pharmingen) per 10⁶ cells were added, the cells were incubated in the dark for at least 20 min. For comparability DNA was counterstained with 7AAD at a final concentration of 10 µg/ml.

### (C) Flow cytometry

All flow cytometric analyses were performed on an EPICS XL MCL (Coulter-Immunotech Diagnostics, Krefeld, Germany) equipped with single air-cooled argon laser with an excitation line at 488nm. Listmode data was acquired and processed with SYSTEM II software (Coulter-Immunotech Diagnostics, Krefeld, Germany) Version 2.1. Illustrations were created using WinMDI Ver. 2.7 (J. Trotter, Salk Institute, USA). Green fluorescence (FITC) was detected through a 525nm band pass filter, orange emission (R-Phycoerythrin) through a 575nm band pass filter and deep red fluorescence from 7AAD was detected through a 675nm band pass filter. Spectral overlap was compensated electronically. All measurements were performed under low sample pressure and at least 10,000 cells of each cell line were measured. BrdUrd sensitivity, described as signal-to-noise ratio (S/N-ratio), was determined by mean fluorescent intensity of mid S-phase cells divided by mean fluorescent intensity of G1 cells.

### (D) Ultraviolet Irradiation

Most standard UV treatments were carried out with an eight Watt UV lamp (HEROLAB, Wiesloch, Germany) emitting predominantly UV-B (280-320nm). Up to 4 × 10⁶ cells were transferred into 14ml tubes, resuspended in 12 ml PBS and placed directly on the lamp's filter surface and irradiated for 5 min. For further clarification of the method's mechanism some samples were irradiated with UV-A (Fenwal®FX-1019 UV Illumination System, Baxter, München, Germany) for 5 min, with a conventional UV Hood lamp emitting mostly UV-C for 5 min or with ultraselective 311 ± 2 nm UV-B (Waldmann UV 1000 cabin, Villingen-Schwennigen, Germany) equipped with ultraselective UV-B bulbs, clinically used for ultraselective phototherapy (SUP) in patients with skin disease) for 5 min. In some experiments cells were irradiated with either UV-A in the presence of 10 µg/ml Hoechst dye 33258 (Sigma, Deisenhofen, Germany) or with UV-B in the presence of 20 µM sodium ascorbate (Sigma) or 20 µM cysteamine (Sigma). Furthermore, a few samples were subjected to 30 Gy Cs¹³⁷ γ-irradiation or 10 mM H₂O₂ incubation to bypass UV-irradiation.

### (E) FIXATION

After UV treatment the cells were centrifuged at 300g for 10 min, the supernatant removed, the pellet resuspended in µl of PBS and the cells were then transferred into Eppendorf® tubes. Immunophenotyping was carried out as described above. If not indicated otherwise, 2 × 10⁶ cells were washed in PBS and fixed in one ml 0.25% PFA/PBS at room temperature (RT) for 15 min. Cells subjected to coagulative fixation were fixed by dropwise addition of cold (-20°C) 70% ethanol and fixed for 45 min at 20°C. After fixation cells were washed once with PBS.

### (F) BrdUrd detection

In all experiments a FITC-conjugated anti-BrdUrd-mAb (clone 3D4, Pharmingen, Hamburg, Germany) was used. Fixed cells were resuspended in 200 µl PBS. 1000 µl Aqua dest. were then added and cells were transferred into flow cytometer tubes. 50 µl of 0.1 M sodium tetraborate (pH 8.0) and 20 µl anti-BrdUrd-FITC (3D4, Pharmingen) were both added to the tubes. DNA was counterstained by adding 7-amino-actinomycin D (7AAD, Sigma) to a final concentration of 10 µg/ml. In some experiments 7AAD was replaced by propidiumiodide (PI, Sigma) with a final concentration of 10 µg/mL. In both cases, cells were incubated at least 60 min (preferable to incubate for 2 hrs) at RT in the dark prior flow cytometric measurement. For immunofluorescence microscopy, cells were prepared as described above and carefully spun down on slides. Cells were visualized with an Axioplan 2 (Zeiss, Jena, Germany).

### Example 2: Ultraviolet-induced detection of S-phase cells

To overcome limitations and the complexity of the traditional S-phase specific cell cycle analysis the method of the present invention was used that generates a sensitive detection of ,e.g. BrdUrd, by ultraviolet induction while maintaining sufficient antigen preservation. The flow cytometric protocol used for the detection of BrdUrd incorporation in S-phase cells in a heterogeneous cell suspension is illustrated in the legend to Fig. 1. The CD19 positive cell line Raji was mixed with tetraploid Molt-4 cells. After treatment according to the methods of Example 1 the immunophenotyping on Raji remained intact, enabling selective analysis of the cell cycle of each cell line. The formation of doublets was low for cells with PFA fixation and aggregates were excluded accurately by gating strategy. S-phase cells were labelled with FITC-conjugated anti-BrdUrd-mAB staining all cells with intermediate DNA content, i.e. between single and double DNA content. Cells in G1 phase and G2/M phase remained unlabelled and did not show uptake of the thymidine analogue. Acid denaturation after UV light exposure revealed that only a part of the BrdUrd is photolysed by irradiation and that sufficient amounts of BrdUrd remain to be detected by the mAB (Figure 2.)

### Example 3: Investigation of the influence of several UV light sources

This experiment was carried out in order to study the influence of several UV light sources with different wavelengths on the sensitivity of BrdUrd detection in HL-60 as described in the legend to Fig. 3. Cells that did not undergo any UV irradiation at all, or were irradiated with UV-A alone showed no or almost no BrdUrd signal, respectively. However, cells containing BrdUrd, that were irradiated with UV-B ,UV-C or UV-A in combination with Hoechst dye 33258 developed a strong green fluorescence. UV-B irradiation generated slightly stronger signals than using UV-C. However, a UV-B contamination of the UV-C source, a conventional Hood lamp, could not be excluded. UV-A plus Hoechst dye 33258 seemed to be just as effective as UV-B. Interestingly, irradiation periods longer than 5 min did not increase sensitivity. To exclusively probe UV-B, light from an ultraselective UV-B source (311±2 nm) was applied leading to a the highest sensitivity. The UV-B lamp from Herolab containing one lightbulb with 8 Watts emitting wavelengths between 280-320 nm, produced reproducible results and was chosen for routine use.

The following conclusion can be drawn from this experiment: Not all wavelengths of UV-light are equally effective in detection of BrdUrd. In the experiment it could be shown that UV-B light, 280-320 nm, was highly efficient in inducing BrdUrd detection. UV-B, in particular wavelengths around 312nm, have been described to effectively photolyze BrdUrd. By debromination uracilyl-radicals are formed that can attack the sugar-phosphate backbone of DNA resulting in SSBs and uracil formation (32). UV-A exerts similar effects only in the presence of Hoechst dyes, binding with high affinity to BrdUrd substituted DNA yielding high amounts of SSBs, DSBs and uracil (38-40). The suggestion that the sensitivity of the method of the invention strongly depends on radical formation and extensive damage to DNA and chromatin is further stressed by the inhibition of BrdUrd detection in the presence of antioxidants such as cysteamine or sodium ascorbate as shown in Fig. 4 confirming recently published data (41, 42).

### Example 4: Investigation of the influence of antioxidants

In this experiment the influence of antioxidants on the sensitivity was investigated. The presence of antioxidants such as cysteamine and sodium ascorbate under UV-B irradiation strongly inhibited BrdUrd detection in HL-60 cells. In addition, replacement of UV-B with the double-strand-break (DSB) and single-strand-break (SSB) inducer γ-radiation (30Gy) or with the SSB inducer H₂O₂ (10 mM) did not lead to BrdUrd detection (Fig. 4).

### Example 5: Investigation of the influence of the ionic strength of the buffer used for resuspending the cells after UV treatment

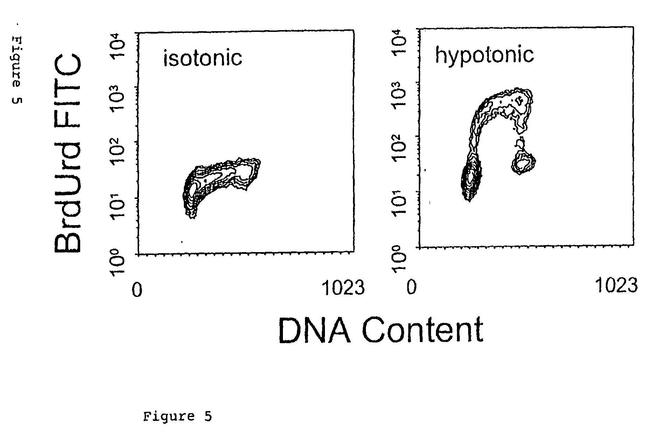

Only UV treated cells in a buffer with reduced ionic strength(hyptonic solution; in this case 68 mosm) were amenable to BrdUrd detection. Cells that were resuspended in isotonic PBS (300 mosm) did not allow antibody binding as shown in Fig. 5. Resuspension in distilled water with 5% glucose (300mosm) revealed that it is not the hypoosmolality itself but the decrease in the ionic background that enables BrdUrd detection (data not shown). In contrast, in DNA that has been physically (e.g. by acid) or enzymatically denatured, the BrdUrd epitope is accessible in isotonic PBS. The data obtained by flow cytometry were confirmed by immunofluorescence microscopy.

To conclude, UV irradiation alone does not allow mAb binding. An environment of reduced ionic strength is needed to alter the structure of damaged DNA and chromatin so that BrdUrd can be detected. The most likely mechanisms are increased chromatin decondensation and partial DNA denaturation by lack of positive-charged counter-ions that neutralize the negatively charged backbone of DNA (49). The change in BrdUrd accessibility by hypotonic treatment remains reversible for a long period of time and the signal decreases upon addition of sodium chloride.

### Example 6: Immofluorescence microscopy of cells prepared according to the method of the invention fixed on slides

Fig.7 shows the applicability of the method of the invention on slides. Raji cells were prepared by the methods described in Example 1 and the legend to Fig.7 and then carefully spun on slides. BrdUrd negative and positive nuclei could be identified. The FITC signal is restricted to the nucleus and strong enough to be detected on the slides.

### Example 7: Detection of cells showing apoptosis

As simultaneous analysis of apoptosis and proliferation is highly desirable in tumor biology (24) HS-Sultan cells were treated according to the methods described in Example 1 and the legend to Fig.8. In order to induce apoptosis, the cells were exposed to 30Gy γ-irradiation 3 hours before the addition of BrdUrd. The cells were incubated for another hour at 37°C. Cells were then harvested, split and either fixed with crosslinking 0.25% PFA for 15min at RT or with 70% ethanol at -20°C for 45 min. Results are illustrated in Fig. 8. The low-molecular-weight DNA (LMW-DNA) characteristic for apoptotic cells could not be extracted after PFA fixation, resulting in a BrdUrd negative fraction with intermediate DNA content. After ethanol fixation ,however, LMW-DNA can be extracted and apoptotic cells can be clearly identified in a sub-G_{0/1} position (25).

### Example 8: Pulse-chase experiments

Pulse-chase experiments are useful in determining cell-cycle kinetics, i.e. cells in S-Phase were pulse labelled with BrdUrd and their progression through the cell cycle was followed by sampling at later stages. It was therefore necessary to test whether detection of BrdUrd by the method of the invention is restricted solely to the S-phase, or if all cell cycle stages, independent of their different chromatin structure, would allow for BrdUrd identification. Samples analyzed 0, 4 and 8 hrs after pulse labeling provided evidence that by using the method of the invention BrdUrd incorporation can be detected in labelled cells independent from their current cell cycle position, making this method suitable for kinetic analyses (Fig. 9). Thus, it is apparent that the combination of UV induced DNA/chromatin damage and hypotonic decondensation enables BrdUrd detection in all cell-cycle compartments and suggests its use in kinetic analyses as well. In combination with surface markers, as in the example with CD138 (B-B4), the method of the invention can be used to determine cell kinetics in multiple myeloma patients providing valuable prognostic information (12-14).

### Example 9: Immunophenotyping

Another important feature of the method of the invention is that surface markers are well enough maintained to allow for the simultaneous analysis of different cell types in heterogeneous cell suspensions. As shown in Fig. 10, three pairs of different cell lines were mixed, surface stained with PE-conjugated mABs, fixed with PFA and subjected to the procedure of the invention. CD34 positive KG1a cells could be discriminated from HL-60 cells, CD138 (B-B4) expressing U266 cells from HS-Sultan cells and CD19 positive Raji cells from Molt-4 cells. All six cell lines could be differentiated by phenotype and analyzed separately.

### Example 10: Correlation of the method of the invention with HCl denaturation

Doublet formation, S-phase fraction, signal-to-noise-ration (S/N-ratio) and the CV of the G0/1 peak in the DNA histogram were compared for both methods. The S-phase fractions (SPF) obtained from three immunophenotyping experiments with the six different cell lines KG1a, HL-60, U266, HS-Sultan, Raji and Molt-4 were correlated with the SPF acquired by classical acid denaturation. The SPF's of both methods correlated very well with a Pearson's correlation coefficient of r = 0.938 (see Fig. 11).

There was a considerably higher amount of doublets from G1 cells with the acid denaturation procedure resulting from ethanol fixation (4.22 ± 1.73%) in comparison to PFA fixed cells treated according to the method of the invention (1.41 ± 0.98%). Due to its good correlation the overall SPF is almost identical (Method of the invention: 50.34 ± 5.18% versus HCl denaturation: 50.75 ± 5.65%). Considering sensitivity in terms of signal-to-noise ratio (S/N-ratio) as defined above, the method of the invention proved to be more sensitive than HCl denaturation (2 N for 20 min at RT). With a S/N-ratio almost twice as high as HCl denaturation (Method of the invention: 16.83 ± 5.85 versus HCl denaturation: 9.00 ± 2.56), the discrimination of S-phase was easier in cells treated according to the method of the invention. However, the coefficients of variation (CV) of the G_{0/1} peak of the DNA histogram were smaller in cells treated with acid denaturation (Method of the invention: 4.27 ± 0.55% versus HCl denaturation: 3.04 ± 0.33%). The data obtained are summarized in Table 1:

**Table 1**

| | **Doublets** | **S-phase** | **S-phase/G0/1** | **CV (G0/1 peak)** |
|---|---|---|---|---|
| Invention | 1.41 ± 0.98% | 50.34 ± 5.18% | 16.83 ± 5,85% | 4.27 ± 0.55% (3.50-5.77) |
| HCl denat. | 4.22 ± 1.73% | 50.75 ± 5.65% | 9.00 ± 2.56 | 3.04 ± 0.33% (2.55-3.86) |

To summarize, the average S/N-ratio of cells treated according to the method of the invention was almost double as high as cells denatured with 2 N HCl at RT providing enough sensitivity for comfortable BrdUrd discrimination. Thus, the method of the invention is sensitive enough to detect BrdUrd on a linear scale as early as 5 min of incubation with 10 µM BrdUrd. After 15 min a comfortable discrimination between labelled and unlabelled cells is feasible. If higher sensitivities are desired, the method of the invention can be combined with indirect antibody staining enhancing the sensitivity about 3-fold resulting in S/N ratios of 60-80 (data not shown).

### List of references

1. Giordano, M., Riccardi, A., Danova, M., Brugnatelli, S., and Mazzini, G. Cell proliferation of human leukemia and solid tumors studied with in vivo bromodeoxyuridine and flow cytometry. Cancer Detect. Prev., 15: 391-396, 1991.
2. Durand, R. E., and Raleigh, J. A. Identification of nonproliferating but viable hypoxic tumor cells in vivo. Cancer Res., *58:* 3547-3550, 1998.
3. Vasseur, F., Le Campion, A., Pavlovitch, J. H., and Penit, C. Distribution of cycling T lymphocytes in blood and lymphoid organs during immune responses. J. Immunol., *162:* 5164-5172, 1999.
4. Dolbeare, F. Bromodeoxyuridine: a diagnostic tool in biology and medicine, Part I: Historical perspectives, histochemical methods and cell kinetics. Histochem. J., *27:* 339-369,1995.
5. Dolbeare, F., Bromodeoxyuridine: a diagnostic tool in biology and medicine, Part II: Oncology, chemotherapy and carcinogenesis. Histochem. J., *27:* 923-964,1995.
6. Dolbeare, F. Bromodeoxyuridine: a diagnostic tool in biology and medicine, Part III: Proliferation in normal, injured and diseased tissue, growth factors, differentiation, DNA replication sites and in situ hybridization. Histochem. J., *28:* 531-575,1996.
7. Begg, A. C., McNally, N. J., Shrieve, D. C., and Karchner, H. A. Method to measure the duration of DNA synthesis and the potential doubling time from a single sample. Cytometry, *6:* 620-626, 1985.
8. White, R. A., Terry, N. H., Baggerly, K. A., and Meistrich, M. L. Measuring cell proliferation by relative movement. I. Introduction and in vitro studies. Cell Prolif. 24: 257-270, 1991.
9. Tinnemans, M. M., Lenders M. H., ten Velde, G. P., Blijham, G. H., Ramaekers, F. C., and Schutte, B. Prognostic value of cytokinetic parameters in lung cancer after in vivo bromodeoxyuridine labeling. Anticancer Res., *19:* 531-534, 1999.
10. Witzig, T. E., Habermann, T. M., Kurtin, P. J., Schroeder, G., Stenson, M. J., and Greipp, P. R. S-phase fraction by the labeling index as a predictive factor for progression and survival in low grade Non-Hodgkin's lymphoma. Cancer, *76:* 1059-1064, 1995.
11. Lamborn, K. R., Prados, M. D., Kaplan, S. B., and Davis, R. L. Final report on the University of California-San Francisco experience with bromodeoxyuridine labeling index as a prognostic factor for the survival of glioma patients. Cancer, *85:* 925-935, 1999.
12. San Miguel, J. F., Garcia-Sanz, R., Gonzalez, M., Moro, M. J., Hernandez, J. M., Ortega, F., Borrego, D., Carnero, M., Casanova, F., and Jiminez, R. A new staging system for multiple myeloma based on the number of S-phase plasma cells. Blood, *85:* 448-455, 1995.
13. Witzig, T. E., Timm, M., Larson, D., Therneau, T., and Greipp, P. R. Measurement of apoptosis and proliferation of bone marrow plasma cells in patients with plasma cell proliferative disorders. Br. J. Haematol., 104: 131-137, 1999.
14. Joshua, D., Petersen, A., Brown, R., Pope, B., Snowdon, L., and Gibson, L. The labeling index of primitive plasma cells determines the clinical behavior of patients with myelomatosis. Br. J. Haematol., *94:* 76-81, 1996.
15. Gratzner, H. G. Monoclonal antibody to 5-bromodeoxyuridine. A new reagent for detection of DNA replication. Science (Washington DC), *218:* 474-475, 1982.
16. Dolbeare, F., Gratzner, H. G., Pallavicini, M. G., and Gray, J. W. Flow cytometric measurement of total DNA content and incorporated bromodeoxyuridine. Proc. Natl. Acad. Sci. USA, *80:* 5573-5577, 1983.
17. Gonchoroff, N. J., Katzmann, J. A., Currie, R. M., Evans, E. L., Houck, D. W., Kline, B. C., Greipp, P. R., and Loken, M. R. S-phase detection with an antibody to bromodeoxyuridine. Role of DNAse pretreatment. J. Immunol. Meth., *93:* 97-101, 1986.
18. Dolbeare, F., and Gray, J. W. Use of restriction endonucleases and exonuclease III to expose halogenated pyrimidines for immunochemical staining. Cytometry, *9:* 631-635, 1988.
19. Bayer, J. A., De Vries, P., Herweijer, H., and Bauman, J. G. The use of E. coli exonuclease III to generate single stranded DNA in BrdUrd cell-cycle analysis permits simultaneous detection of cell surface antigens. J. Immunol. Meth., *132:* 13-24, 1990.
20. Carayon, P., and Bord, A. Identification of DNA replicating lymphocyte subsets using a new method to label the bromo-deoxyuridine incorporated into the DNA. J. lmmunol. Meth., 147: 225-230, 1992.
21. Li, X., Traganos, F., Melamed, M. R., and Darzynkiewicz, Z. Detection of 5-bromo-2-deoxyuridine incorporated into DNA by labeling strand breaks induced by photolysis (SBIP). Int. J. Oncol., 4: 1157-1161, 1994.
22. Penit, C., and Vasseur, F. Phenotype analysis of cycling and postcycling thymocytes: Evaluation of detection methods for BrdUrd and surface proteins. Cytometry, *14:* 757-763, 1993.
23. Takagi, S., McFadden, M. L., Humphreys, R. E., Woda, B. A., and Sairenji, T. Detection of 5-bromo-2-deoxyuridine (BrdUrd) incorporation with monoclonal anti-BrdUrd antibody after deoxyribonuclease treatment. Cytometry, *14:* 640-648, 1993.
24. Li, X., Traganos, F., and Darzynkiewicz, Z. Simultaneous analysis of DNA replication and apoptosis during treatment of HL-60 cells with camptothecin and hyperthermia and mitogen stimulation of human lymphocytes. Cancer Res., *54:* 4289-4293, 1994.
25. Darzynkiewicz, Z., Bruno, S., Del Bino, G., Gorczyca, W., Hotz, M. A., Lassota, P., and Traganos, F. Features of apoptotic cells measured by flow cytometry. Cytometry, *13:* 795-808, 1992.
26. Latt, S. A. Microfluorimetric detection of deoxynucleic acid replication in human metaphase chromosomes. Proc. Natl. Acad. Sci. USA, *70:* 3395-3399, 1973.
27. Latt, S. A. Detection of DNA synthesis in interphase nuclei by fluorescence microscopy. J. Cell. Biol., *62:* 546-550, 1974.
28. Darzynkiewicz, Z., Traganos, F., and Melamed, M. R. Distinction between 5-bromodeoxyuridine labelled and unlabelled mitotic cells by flow cytometry. Cytometry, *3:* 345-348, 1983.
29. Kubbies, M., and Rabinovitch, P. S. Flow cytometric analysis of factors which influence the BrdUrd-Hoechst quenching effect in cultivated human fibroblasts and lymphocytes. Cytometry, *3:* 276-281, 1983.
30. Poot, M., Kubbies, M., Hoehn, H., Grossman, A., Chen, Y., and Rabinovitch, P. Cell cycle analysis using continuous bromodeoxyuridine labeling and Hoechst 33258-ethidium bromide bivariate flow cytometry. Meth. Cell. Biol., *33:* 185-198, 1990.
31. Crissman, H. A. and Steinkamp, J. A. A new method for rapid and sensitive detection of bromodeoxyuridine in DNA-replicating cells. Exp. Cell Res., 173: 256-261, 1987.
32. Hutchinson, F. The lesions produced by ultraviolet light in DNA containing 5-bromouracil. Quart. Rev. Biophys., *6:* 201-246, 1973.
33. Ogata, R., and Gilbert, W. Contacts between the lac repressor and the thymines in the lac operator. Proc. Natl. Acad. Sci. USA *74:* 4973-4976, 1977.
34. Murray, V., and Martin, R. F. The degree of ultraviolet light damage to DNA containing iododeoxyuridine or bromodeoxyuridine is dependent on the DNA sequence. Nucleic Acids Res., *17):* 2671-91, 1989.
35. Sugiyama, H., Fujimoto, K., Hatano, K., Ohmori, K., and Saito, I. Chemistry of oxidative DNA strand scission. Nucleic Acids Symp. Ser., 29: 125-126, 1993.
36. Fujimoto, K., Sugiyama, H., and Saito, I. Photoreaction of 5-bromouracil-containing DNA. Nucleic Acids Symp. Ser., *34:* 25-26, 1995.
37. Fujimoto, K., Ishihara, S., and Saito, I. Mechanistic investigation of photoreduction of 5-bromouracil-containing oligomers. Nucleic Acids Symp. Ser., *37:* 89-90, 1997.
38. Limoli, C. L., and Ward, J. F. A new method for introducing double-strand breaks into cellular DNA. Radiat. Res., *134(2):* 160-169, 1993.
39. Limoli, C. L., and Ward, J. F. DNA damage in bromodeoxyuridine substituted SV40 DNA and minichromosomes following UVA irradiation in the presence of Hoechst dye 33258. Int. J. Radiat. Biol., *66:* 717-728, 1994.
40. Limoli, C. L., Wu, C. C., Milligan, J. R., and Ward, J. F. Photochemical production of uracil quantified on bromodeoxyuridine-substituted SV40 DNA by uracil DNA glycosylase and a lysyl-tyrosyl-lysine tripeptide. Mutagenesis, *12:* 443-447, 1997.
41. Bianchi, M., Bianchi, N., Cortes, L., and Reigosa, M. Cysteamine protection of SCEs induced by UV and fluorescent light. Mutat. Res., *104:* 281-286, 1982.
42. Murray, D., Prager, A., Vanankeren, S. C., Altschuler, E. M., Kerr, M. S., Terry, N. H., and Milas, L. Comparative effect of the thiols dithiothreitol, cysteamine and WR-151326 on survival and on the induction of DNA damage in cultured Chinese hamster ovary cells exposed to gamma-radiation. Int. J. Radiat. Biol., *58(1):* 71-91, 1990.
43. Webb, C. F., Jones, G. D., Ward, J. F., Moyer, D. J., Aguilera, J. A., and Ling, L. L. Mechanisms of radiosensitization in bromodeoxyuridine-substituted cells. Int. J. Radiat. Biol., *64(6):* 695-705, 1993.
44. Fuciarelli, A. F., Sisk, E. C., Miller, J. H., and Zimbrick, J. D. Radiation-induced electron migration in nucleic acids. Int. J. Radiat. Biol., *66(5):* 505-509, 1994.
45. Mello Filho, A. C., and Meneghini, R. In vivo formation of single-strand breaks in DNA by hydrogen peroxide is mediated by the Haber-Weiss reaction. Biochim. Biophys. Acta., *781(1-2):* 56-63, 1984.
46. Taichman, L. B. The use of ultraviolet light in the fractionation of chromatin containing unsubstituted and bromodeoxyuridine-substituted DNA. Nucleic Acids Res., *6(5):* 2029-2038, 1979.
47. Zwanenburg, T. S., van Zeeland, A. A., and Natarajan, A. T. Influence of incorporated bromodeoxyuridine on the induction of chromosomal alterations by ionizing radiation and long-wave UV in CHO cells. Mutat. Res., *150:* 283-292, 1985.
48. Mezzanotte, R., Peretti, D., Orru, S., Rossino, R., Ennas, M. G., and Gosalvez, J. DNA alteration induced by ultraviolet light in human metaphase chromosomes substituted with 5'-bromodeoxy uridine: monitoring by monoclonal antibodies to double-stranded and single-stranded DNA. Chromosoma, *97:* 356-362, 1989.
49. Van Holde, K., and Zlatanova, J. What determines the folding of the chromatin fiber ? Proc. Natl. Acad. Sci. U.S.A., *93:* 10548-10555, 1996.
50. Zelenin, A. V., Poletaev, A. I., Stapanova N. G., Barsky, V. E., Kolesnikov, V. A., Nikitin, S. M., Zhuze, A. L., and Gnutchev, N. V. 7 amino-actinomycin D as a specific fluorophore for DNA content analyses by laser flow cytometry. Cytometry, 5: 348-354, 1984.
51. Rabinovitch, P. S., Torres, R. M., and Engel, D. Simultaneous cell cycle analysis and two-color surface immunoflourescence using 7-aminoactinomycin D and single laser excitation: Applications to the study of cell activation and the cell cycle of murine LY-1 B cells. J. Immunol., 136: 2769-2775, 1986.
52. Shapiro, H. M. Parameters and probes. In: H. M. Shapiro (ed.), Practical Flow Cytometry, pp. 130-132. New York: Wiley-Liss, 3rd edition 1994
53. Holm, M., Thomsen, M., Hoyer, M., and Hokland, P. Optimization of a flow cytometric method for the simultaneous measurement of cell surface antigen, DNA content, and in vitro BrdUrd incorporation into normal and malignant hematopoietic cells. Cytometry, *32:* 28-36, 1998.

## Claims

1. A method for identifying DNA replicating cells, which method comprises the following steps:
(a) adding during the cultivation of cells a halogenated pyrimidine to the medium;
(b) treating the harvested cells by UV irradiation;
(c) fixing the cells prior to or after UV irradiation;
(d) resuspending the cells in a hypotonic solution having an osmolarity of less than 200 mosm; and
(e) identifying cells with incorporated halogenated pyrimidine.

2. The method of claim 1, wherein the halogenated pyrimidine is a halogenated thymidine analogue.

3. The method of claim 2, wherein the halogenated thymidine analogue is 5-bromo-2-deoxyuridine (BrdUrd), 5-iodo-2-deoxyuridine (IdUrd), 5-fluoro-2-deoxyuridine (FdUrd) or 5-chloro-2-deoxyuridine (CldUrd).

4. The method of any one of claims 1 to 3, wherein the irradiation is carried out with UV-A, UV-B or UV-C light.

5. The method of claim 4, wherein the irradiation is carried out with UV-B light with a wavelength in the range between 280 to 320 nm.

6. The method of claim 5, wherein the wavelength of the UV-B light is in the range between 309 to 313 nm.

7. The method of any one of claims 1 to 6, wherein the osmolarity of the solution with reduced ionic strength is in the range between 50 and 100 mosm.

8. The method of any one of claims 1 to 7, wherein the cells are identified with either labelled or unlabelled monoclonal or polyclonal antibodies specific for the halogenated pyrimidine.

9. The method of claim 8, wherein the antibody is anti-BrdUrd.

10. The method of any one of claims 1 to 9, wherein the cells are identified by flow cytometry, immunofluorescence microscopy, confocal microscopy or laser-scan microscopy.

11. The method of any one of claims 1 to 10, further comprising the simultaneous detection of at least one cellular marker.

12. The method of claim 11, wherein the detection of the cellular marker is by immunophenotyping of surface antigens, immunophenotyping of cytoskeletal proteins or other intracellular antigens or by detection of apoptosis.

13. The method of any one of claims of 1 to 12 for the diagnosis or prognosis of a malignancy, benign disease, immunological disorder or infectious disease.

14. The method of any one of claims 1 to 13 for analysing apoptosis, proliferation or cell-cycle kinetics.

## Patentansprüche

1. Verfahren zum Identifizieren von DNA-replizierenden Zellen, wobei das Verfahren die folgenden Schritte umfaßt:
(a) Zugeben eines halogenierten Pyrimidins zum Medium während des Züchtens von Zellen;
(b) Behandeln der geernteten Zellen durch UV-Bestrahlung;
(c) Fixieren der Zellen vor oder nach der UV- Bestrahlung;
(d) Resuspendieren der Zellen in einer hypotonischen Lösung mit einer Osmolarität von weniger als 200 mosm; und
(e) Identifizieren von Zellen mit einem eingebauten halogenierten Pyrimidin.

2. Verfahren nach Anspruch 1, wobei das halogenierte Pyrimidin ein halogeniertes Thymidin-Analog ist.

3. Verfahren nach Anspruch 2, wobei das halogenierte Thymidin-Anlog 5-Brom-2-desoxyuridin (BrdUrd), 5-Iod-2-desoxyuridin (IdUrd), 5-Fluor-2-desoxyuridin (FdUrd) oder 5-Chlor-2-desoxyuridin (CldUrd) ist.

4. Verfahren nach einem der Ansprüche 1 bis 3, wobei die Bestrahlung mit UV-A, UV-B oder UV-C-Licht durchgeführt wird.

5. Verfahren nach Anspruch 4, wobei die Bestrahlung mit UV-B-Licht mit einer Wellenlänge im Bereich zwischen 280 bis 320 nm durchgeführt wird.

6. Verfahren nach Anspruch 5, wobei die Wellenlänge des UV-B-Lichts im Bereich zwischen 309 bis 313 nm ist.

7. Verfahren nach einem der Ansprüche 1 bis 6, wobei die Osmolarität der Lösung mit reduzierter Ionenstärke im Bereich zwischen 50 und 100 mosm ist.

8. Verfahren nach einem der Ansprüche 1 bis 7, wobei die Zellen entweder mit markierten oder unmarkierten monoclonalen oder polyclonalen Antikörpern identifiziert werden, die spezifisch für das halogenierte Pyrimidin sind.

9. Verfahren nach Anspruch 8, wobei der Antikörper Anti-BrdUrd ist.

10. Verfahren nach einem der Ansprüche 1 bis 9, wobei die Zellen mittels Durchflusscytometrie, Immunfluoreszenz-Mikroskopie, konfokaler Mikroskopie oder Laserscan-Mikroskopie identifiziert werden.

11. Verfahren nach einem der Ansprüche 1 bis 10, zusätzlich umfassend den gleichzeitigen Nachweis von mindestens einem zellulären Marker.

12. Verfahren nach Anspruch 11, wobei der Nachweis des zellulären Markers durch Immunphänotypisierung eines Oberflächenantigens, Immunphänotypisierung eines Cytoskelettproteins oder anderer intrazellulärer Antigene oder durch den Nachweis von Apoptose erfolgt.

13. Verfahren nach einem der Ansprüche 1 bis 12 zur Diagnose oder Prognose einer bösartigen Krankheit, gutartigen Krankheit, immunologischen Störung oder infektiösen Krankheit.

14. Verfahren nach einem der Ansprüche 1 bis 13, zum Analysieren von Apoptose, Proliferation oder Zellzykluskinetiken.

## Revendications

1. Procédé pour identifier des cellules réplicant l'ADN, laquelle méthode comprend les étapes suivantes :
(a) ajouter une pyrimidine halogénée au milieu pendant la culture des cellules ;
(b) traiter les cellules récoltées par irradiation UV ;
(c) fixer les cellules avant ou après irradiation UV ;
(d) remettre en suspension les cellules dans une solution hypotonique ayant une osmolarité de moins de 200 mosm ; et
(e) identifier les cellules ayant incorporé la pyrimidine halogénée.

2. Procédé selon la revendication 1, dans lequel la pyrimidine halogénée est un analogue halogéné de la thymidine.

3. Procédé selon la revendication 2, dans lequel l'analogue halogéné de la thymidine est la 5-bromo-2-désoxyuridine (BrdUrd). la 5-iodo-2-désoxyuridine (IdUrd). la 5-fluoro-2-désoxyuridine (FdUrd) ou la 5-chloro-2-désoxyuridine (CldUrd).

4. Procédé selon l'une quelconque des revendications 1 à 3. dans lequel l'irradiation est réalisée avec une lumière UV-A, UV-B ou UV-C.

5. Procédé selon la revendication 4, dans lequel l'irradiation est réalisée avec une lumière UV-B avec unc longueur d'onde dans l'intervalle entre 280 à 320 nm.

6. Procédé selon la revendication 5, dans lequel la longueur d'onde de la lumière UV-B est dans l'intervalle entre 309 à 313 nm.

7. Procédé selon l'une quelconque des revendications 1 à 6, dans lequel l'osmolarité de la solution ayant une force ionique réduite est dans l'intervalle entre 50 et 100 mosm.

8. Procédé selon l'une quelconque des revendications 1 à 7, dans lequel les cellules sont identifiées avec des anticorps monoclonaux ou polyclonaux soit marqués, soit non marqués, spécifiques de la pyrimidine halogénée.

9. Procédé selon la revendication 8, dans lequel l'anticorps est un anti-BrdUrd.

10. Procédé selon l'une quelconque des revendications 1 à 9, dans lequel les cellules sont identifiées par cytométrie en flux, microscopie par immunofluorescence, microscopie confocale ou microscopie laser à balayage.

11. Procédé selon l'une quelconque des revendications 1 à 10, comprenant en outre la détection simultanée d'au moins un marqueur cellulaire.

12. Procédé selon la revendication 11, dans lequel la détection du marqueur cellulaire se fait par immunophénotypage d'antigènes de surface immunophénotypage de protéines du cytosquelette ou d'autres antigènes intracellulaires ou par détection de l'apoptose.

13. Procédé selon l'une quelconque des revendications 1 à 12 pour le diagnostique ou le pronostique d'une malignité, d'une maladie bénigne, d'un désordre immunologique ou d'une maladie infectieuse.

14. Procédé selon l'une quelconque des revendications 1 à 13 pour analyser l'apoptose, la prolifération ou la cinétique du cycle cellulaire.
